(19) Europäisches Patentamt / European Patent Office / Office européen des brevets

(11) **EP 2 532 752 B1**

(12) **EUROPEAN PATENT SPECIFICATION**

(45) Date of publication and mention of the grant of the patent:
**25.05.2016 Bulletin 2016/21**

(51) Int Cl.:
*C12Q 1/02* $^{(2006.01)}$    *G01N 33/24* $^{(2006.01)}$

(21) Application number: **10844619.6**

(22) Date of filing: **01.02.2010**

(86) International application number:
**PCT/JP2010/051329**

(87) International publication number:
**WO 2011/092860 (04.08.2011 Gazette 2011/31)**

(54) **METHOD FOR MEASURING CROP CULTIVATION FREQUENCY OF SOIL AND METHOD FOR ASSESSING PRODUCTION REGION DECEPTION**

VERFAHREN ZUR MESSUNG DER BODENKULTIVIERUNGSFREQUENZ UND VERFAHREN ZUR BEURTEILUNG VON PRODUKTIONSBEREICHSTÄUSCHUNG

MÉTHODE PERMETTANT DE MESURER LA FRÉQUENCE DES CULTURES FOURRAGÈRES D'UN SOL ET MÉTHODE PERMETTANT D'ÉVALUER UNE IMPOSTURE DANS UNE RÉGION DE PRODUCTION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**12.12.2012 Bulletin 2012/50**

(73) Proprietor: **DGC Technology Inc.**
**Tsukuba-shi**
**Ibaraki 3002667 (JP)**

(72) Inventor: **Hatano Shoji**
**Komaki-shi**
**Aichi 485-0012 (JP)**

(74) Representative: **Betten & Resch**
**Patent- und Rechtsanwälte PartGmbB**
**Maximiliansplatz 14**
**80333 München (DE)**

(56) References cited:
**JP-A- 2006 329 639   JP-A- 2007 000 138**
**JP-A- 2010 046 020**

- H Yao ET AL: "Microbial Biomass and Community Structure in a Sequence of Soils with Increasing Fertility and Changing Land Use", Microbial Ecology, vol. 40 1 October 2000 (2000-10-01), pages 223-237, XP055089027, Berlin/Heidelberg DOI: 10.1007/s002480000053 Retrieved from the Internet: URL:http://www.jstor.org/stable/4251772
- CRECCHIO C ET AL: "Functional and molecular responses of soil microbial communities under differing soil management practices", SOIL BIOLOGY AND BIOCHEMISTRY, PERGAMON, OXFORD, GB, vol. 36, no. 11, 1 November 2004 (2004-11-01), pages 1873-1883, XP004565355, ISSN: 0038-0717, DOI: 10.1016/J.SOILBIO.2004.05.008
- CAMPBELL, C. D. ET AL.: 'A Rapid Microtiter Plate Method To Measure Carbon Dioxide Evolved from Carbon Substrate Amendments so as To Determine the Physiological Profiles of Soil Microbial Communities by Using Whole Soil' APPLIED AND ENVIRONMENTAL MICROBIOLOGY vol. 69, no. 6, 2003, pages 3593 - 3599, XP008164759
- GARLAND, J. L. ET AL.: 'Classification and Characterization of Heterotrophic Microbial Communities on the Basis of Patterns of Community-Level Sole-Carbon-Source Utilization' APPLIED AND ENVIRONMENTAL MICROBIOLOGY vol. 57, no. 8, 1991, pages 2351 - 2359, XP008164760

- WIDMER, F. ET AL.: 'Assessing soil biological characteristics: a comparison of bulk soil community DNA-, PLFA-, and BiologTM-analyses' SOIL BIOLOGY AND BIOCHEMISTRY vol. 33, no. 7-8, 2001, pages 1029 - 1036, XP008164761

- SATO, NOBUYOSHI ET AL.: 'Distributed Geographical Origin Identification System for Green-groceries by Trace Element Compositions' TRANSACTIONS OF INFORMATION PROCESSING SOCIETY OF JAPAN vol. 47, no. 7, 15 July 2006, pages 2151 - 2159, XP008164764

**Description**

BACKGROND OF THE INVENTION

Field of the Invention

The present invention relates to a method for measuring whether or not soil is suitable for cultivation of crops by means of testing a diversity of microorganisms that inhabit the soil and a method for determining the authenticity of the production region of an agricultural product by means of comparing a diversity of microorganisms that inhabit the soil adhering to the agricultural product with that from the land indicated as the production region of such agricultural product.

Description of the Related Art

Conventionally, whether or not soil is healthy has been determined based on whether or not disease damage took place in the process of growth following the actual planting of crops. Thus, responses, such as pesticide spraying and the like, following occurrence of disease damage, were made. However, responses such as pesticide spraying and the like, cause soil failure, which has been problematic.

Additionally, as a method for predicting the occurrence of disease damage prior to the planting of crops, a method for estimation based on investigations of occurrence of disease damage in the past and the like was undertaken. However, it was impossible to simply and promptly undertake relevant determinations because previous data for several years were required in order to increase the accuracy of prediction and the like. Furthermore, sudden disease damage was caused in soil that had been healthy until the previous year in some cases. As a result, it was difficult to predict the health condition of soil in advance. Therefore, in recent years, methods for determining the degree of health of soil in a scientific manner have been developed and put into practical use.

Methods for determining the degree of health of soil in a scientific manner include chemical diagnosis methods that analyze cation exchange capacity, phosphate absorption coefficient, pH, soil nutrient content in the soil and the like, and physical diagnosis methods that analyze soil hardness, porosity, moisture retentivity, and the like. However, the aforementioned methods are limited to a one-sided assessment of the nature of soil, and thus, it was necessary to concurrently use multiple soil diagnosis methods in order to realize more multifaceted diagnosis. Additionally, it was difficult to conduct a simplified and prompt diagnosis, and thus dispensing and filtering operations were required for extracts of substances subject to analysis.

As a means of solving the aforementioned problems, a method for analyzing environmental DNA(eDNA) and investigating and diagnosing a diversity of microorganisms in the soil was developed.

Background Art

Patent Literature

Patent literature 1: Kokai (unexamined patent publication) No. 2007-000138
Patent literature 2 : Kokai (unexamined patent publication) No. 2006-329639

YAO, H. ET AL: "Microbial Biomass and Community Structure in a Sequence of Soils with Increasing Fertility and Changing Land Use", Microbial Ecology, vol. 40 1 October 2000 (2000-10-01), pages 223-237, Berlin/Heidelberg, discloses investigating the microbial biomass and community structure in eight Chinese red soils with different fertility and land use history. Two community-based microbiological measurements were used to investigate the microbial ecology of these soils and to determine how land use alters microbial community structure, namely community level physiological profiling (CLPP) using BIOLOG® sole C-source utilization tests, and phospholipid fatty acid (PLFA) profiles. It is found therein that PLFA analysis was a better method than CLPP for assessing broad-spectrum community differences and at the same time attempting to correlate changes with soil fertility.

A method for identifying the geographical original of green-groceries using trace element compositions is disclosed in SATO, NOBUYOSHI ET AL.: "Distributed Geographical Origin Identification System for Green-groceries by Trace Element Compositions", TRANSACTIONS OF INFORMATION PROCESSING SOCIETY OF JAPAN, vol. 47, no. 7, 15 July 2006 (2006-07-15), pages 2151-2159.

SUMMARY OF THE INVENTION

Problems to be Solved by the Invention

Even upon use of the aforementioned methods, it is necessary to abstract and analyze DNA. Therefore, devices and knowledge specialized for such use were required. As a result, it was difficult to conduct a simplified and prompt diagnosis.

Means for Solving the Problems

The present invention is defined in independent claim 1.
A method for diagnosis of soils employs a biological method using the microorganisms that inhabit soil subject to diagnosis. Such method is based on the fact that the more diversified the microorganisms are, the more fertile the corresponding soil is. Diversification of the microorganisms in a specific soil is diagnosed based on the following method. First, the suspension of the soil to be analyzed is added dropwise to a plurality of nutrient sources, the rates of consumption of least some of which differ depending upon microbial species, and the cumulative consumption quantity of the nutrient sources consumed by the microorganisms within a certain period is observed. The reason why "a plurality of nutrient sources, the rates of consumption of least some of which differ depending upon microbial specie" is used is a simple consequence of the effect whereby the more diversified the microorganisms are, the more diversified nutrient sources can be consumed. The present invention further computes the results measured above as "crop cultivation frequency," and thereby, suitability for soil crop cultivation is clearly indicated and usability is enhanced.
Crop cultivation frequency for soil of a production region that is a target of production region deception for agricultural products is checked in advance, crop cultivation frequency for agricultural products labeled as coming from the production region is checked in the same manner, and such frequency is compared with that related to the aforementioned soil of the target production region. Thereby, deception regarding production region is evaluated.
The percentages of different nutrient sources of characteristic values that show the tendency of the cumulative consumption quantity of nutrient sources computed for each nutrient source in lieu of comparing the crop cultivation frequencies of soils are compared. Thereby, deception of production regions can be evaluated more accurately.

ADVANTAGEOUS EFFECT OF THE INVENTION

It is possible to objectively and comprehensively judge the soil environment by using the microorganisms that inhabit the soil. Additionally, it is possible to conduct a more highly accurate and remarkably simplified diagnosis. Therefore, flexible responses to the degree of accuracy demanded by users can be made.
It is possible to determine deception regarding a production region of agricultural products in a simplified method.

BRIEF DESRIPTION OF THE DRAWINGS

Fig. 1 is a conceptual diagram of the first aspect of the present invention.
Fig.2 is a procedural flow chart of the first aspect of the present invention.
Fig.3A is a flow chart of a first example using a regression characteristic value average method for the first aspect of the present invention.
Fig.3B is a flow chart of a second example using a regression characteristic value average method for the first aspect of the present invention.
Fig.4A is a flow chart of a first example using an average data regression method for the first aspect of the present invention.
Fig.4B is a flow chart of a second example using an average data regression method for the first aspect of the present invention.
Fig.5A is a flow chart of a first example using a simplified method for the first aspect of the present invention.
Fig.5B is a flow chart of a first example using a simplified method for the first aspect of the present invention.
Fig.6 is a diagram of results for measuring crop cultivation frequency of soil when using a Gompertz curve as a regression function.
Fig.7 is a diagram of results for measuring crop cultivation frequency of soil when using "y = ax + b" as a regression function.
Fig.8 is a diagram of results for measuring crop cultivation frequency of soil when using "y = ax" as a regression function.

Fig.9 is a diagram of results for measuring crop cultivation frequency of soil when using "y = b" as a regression function.
Fig.10 is a schematic diagram of the second aspect of the present invention.
Fig.11 is a flow chart of the second aspect of the present invention.
Fig.12 is a flow chart of the third aspect of the present invention.

DETAILED DESCRIPTIONS OF PREFERRED EMBODIMENTS

The first embodiment relates to the first aspect of the present invention. The second embodiment relates to the second aspect of the present invention. The third embodiment relates to the third aspect of the present invention. Additionally, the present invention is not at all limited by the following embodiments, and can be implemented in various manners without deviating from scope of the purpose of use thereof.

FIRST EMBODIMENT

<Outline>

The embodiment of the method for measuring crop cultivation frequency of soil is explained hereinafter. Such method allows observation of cumulative quantity of various nutrient sources consumed by microorganisms that inhabit the soil, and thereby, measurement of the diversity of microbial species in such soil. The numeric values indicating the degree of such diversity correspond to frequency, representing the suitability of crop cultivation in such soil.

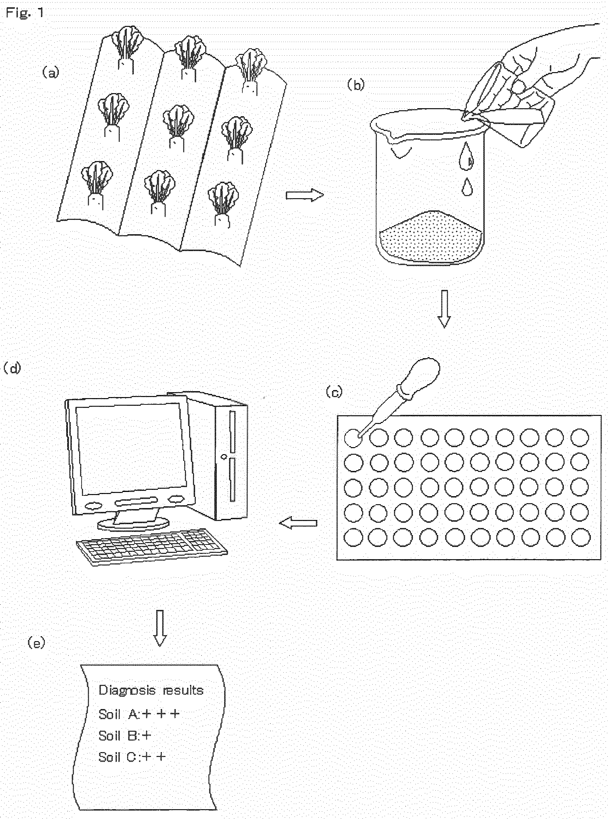

Fig. 1 is a conceptual diagram that shows the method for measuring crop cultivation frequency of soil related to the present invention. For the method for measuring crop cultivation frequency of soil related to the present invention, the soil to be analyzed is first obtained (phase (a)). The obtained soil is sieved, and the resultant is suspended in buffer solution (phase (b)).The suspension is added dropwise to media to which different nutrient sources have been added (phase (c)). Such media is cultured for a predetermined duration and the cumulative consumption quantity of each nutrient source is observed (phase (d)). Based on the cumulative consumption quantity of each nutrient source, the amount and diversity of microorganisms that inhabit the soil to be analyzed are predicted, and the soil crop cultivation frequency is measured (phase (e)).

<Configuration>

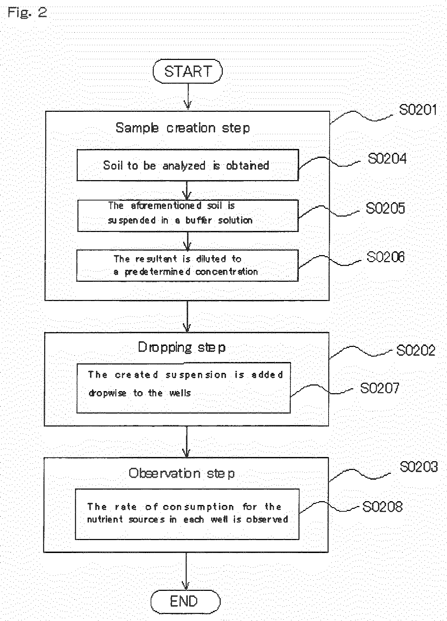

Fig. 2 is a flow chart that shows the method for measuring crop cultivation frequency of soil of the present embodiment. The method for measuring crop cultivation frequency of soil related to the present embodiment comprises a sample creation step (0201), a dropping step (0202), and an observation step (0203). Each step is explained in detail hereinafter.
The sample creation step (0201) "creates a suspension of the soil to be analyzed as a microorganism sample." Soil to be analyzed is obtained (0204) and suspended in a buffer solution (0205), and the resultant is diluted to a predetermined concentration (0206).
The term "soil(s) to be analyzed" refers to soil that is a subject of measurement of crop cultivation frequency. Soil to be obtained may be obtained from a single location of the soil to be analyzed. In order to acquire more highly precise measurement results, such soil may be obtained from a plurality of places that are located relatively far away from each other. For instance, in regards to soil that has been obtained near roads and the soil that has been obtained in the central location of soil to be analyzed, there may exist microbial species that vary depending on surrounding environment in some cases. Therefore, it is preferable to obtain the relevant soil from a plurality of locations giving consideration to the aforementioned matters.
It is desirable to remove stones, grass, trash, and the like by sieving the obtained soil. The scale spacing for sifting is preferably 2 mm. Alternatively, 4 mm is acceptable. In case that the soil cannot not pass through 4 mm sieving, such soil may be used as it is without being sieved. Soil containing a high moisture content may be used after such soil has been slightly dried in the shade.
The obtained soil is suspended in buffer solution, such as a phosphate buffer solution (sterile), and the resultant creates a "suspension" containing microorganisms that inhabit the soil to be analyzed. The created suspension is diluted to a predetermined concentration with sterile water or phosphate buffer solution, and the "microorganism samples" are adjusted. Since the dilution of the concentration will have a direct influence on the amount of consumption of the nutrient sources by microorganisms, special attention is needed. That is to say, when concentration is thinly diluted, the microorganisms in the suspension will decrease. Thus, the rate of consumption of the nutrient sources will become lower. On the other hand, when concentration is thickly diluted, the microorganisms in the suspension will increase. Thus, the rate of consumption of the nutrient sources will become higher. Accordingly, meticulous care is paid so that a specific amount of suspension can be obtained from a specific amount of soil to be analyzed. In particular, care should be taken not to vary the dilution of the concentration among soils to be analyzed.

The "dropping step (0202)" is the step in which "the created suspension as a microorganism sample is added dropwise to a plurality of nutrient sources for at least some of which the rate of consumption differs depending upon microbial species (0207)."

Nutrient sources such as carbon sources, nitrogen sources, phosphorus sources, and potassium sources are effective. However, any nutrient source is acceptable, as long as such source allows nutrient consumption by microorganisms and the rate of consumption differs depending upon microbial species. A plurality of types of carbon sources alone may be used, and it may be acceptable to concurrently use a nitrogen source, a phosphorus source, a potassium source, or other nutrient sources. In order to acquire more highly accurate measurement results, it is preferable to use more types of nutrient sources. In order to acquire a more simplified and prompt measurement results, it is preferable to use only a carbon source. Use of such sources that are appropriate and relevant may be determined based on the measurement accuracy, purpose of measurement, and the like desired by users. A constant amount of suspension to be added dropwise is necessary. In particular, it should be noted that amounts to be added dropwise among the soils to be tested must not vary.

The "observation step (0203)" is the step of "observing cumulative consumption quantity of various nutrient sources consumed by the microorganisms after dropping

(0208)." That is to say, the microorganisms that inhabit the samples in each well are cultured for a certain amount of time and cumulative consumption quantity of nutrient sources to be consumed accompanying microorganism proliferation is observed.

The term "cumulative consumption quantity" refers to the cumulative consumption quantity immediately following commencement of culturing of the nutrient sources until the predetermined time. Additionally, the cumulative consumption quantity may be directly computed by measuring of the remaining nutrient sources within certain hours. In addition, the cumulative consumption quantity may be indirectly observed based on changes in oxidation-reduction ability, turbidity, respiratory volume (carbon dioxide emissions), coloring of bacterial cells by pigment, and intensity of fluorescent staining, all of which depend upon the amounts of microorganisms in culture media. Additionally, the aforementioned methods may be combined. With the latter method, for example, oxidation-reduction coloring reagents that generate color based on the microorganisms' metabolic reactions as well as the nutrient sources are added to the culture media, and changes in color optical density are observed. In case that a more simplified soil diagnosis is desired, the degree of coloring may be judged visually. In case that a more highly accurate soil diagnosis is desired, detailed measurement may be conducted, such as quantifying of the degree of coloring, etc., thereby, allowing observation of cumulative consumption quantity.

The term "certain hours" refers to the length of time for observation of the cumulative consumption quantity mentioned above, and about 48 hours can be established as a rough standard. Also, a necessary and sufficient length of hours that allows differences in consumption quantity among microorganisms to be observed should apply.

The appropriate observation conditions regarding cumulative consumption quantity-that is, the interval of measurement time, the number of points for measurement, and the like-may be determined based on the users' desired measurement accuracy, purpose of measurement, and the soil to be analyzed. Crop cultivation frequency is determined in relative terms. That is to say, the aforementioned observation is conducted for various soils, relative comparison of such results is made, and the crop cultivation frequencies of such soils are judged. Therefore, observation conditions regarding cumulative consumption quantity are required to be the same for all of soils to be analyzed as mentioned above.

The "crop cultivation frequency" is measured based on the cumulative consumption quantity within certain hours observed as described above. More specifically, the crop cultivation frequency is measured via regression analysis through values computed each time cumulative consumption quantity is acquired as a result of observation of the consumption quantity of nutrient sources within certain hours in the soils to be analyzed. The reason for using regression analysis is that a single function under the condition that elapsed time is the explanatory variable and the cumulative consumption quantity is the objective variable is extracted. And a certain trend related to the rate of consumption for the nutrient sources by the microorganisms will be represented by a numeric value at a specific time expressed by the aforementioned function.

<Specific Methods of Measurement of Crop Cultivation Frequency>

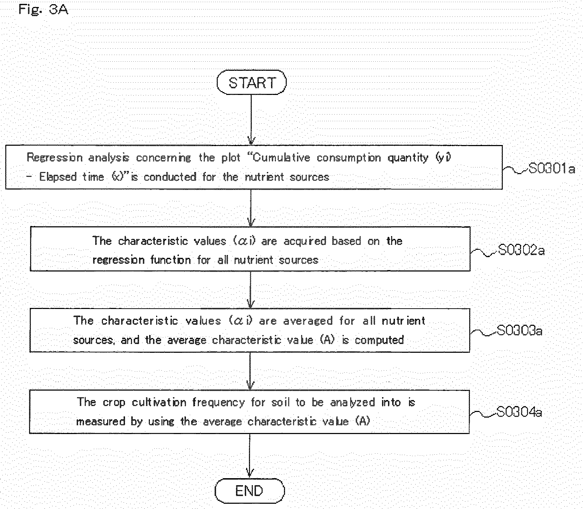

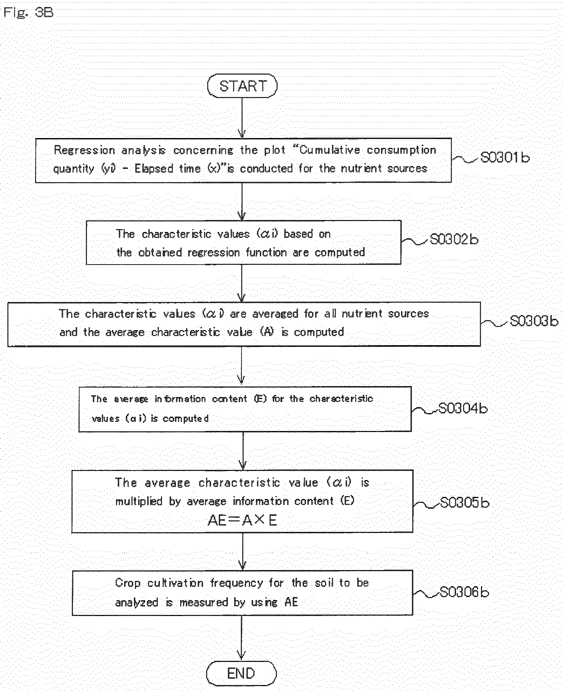

Three specific methods of measurement of crop cultivation frequency are explained using Fig. 3 through Fig. 5. The first method is a regression characteristic value average method, the second method is an average data regression method, and the third med is a simplified method that does not use regression analysis. In addition, one of the methods described in the aforementioned specific examples may be used. More than 2 such methods may be used in combination.

1. Regression Characteristic Value Average Method

The term "regression characteristic value average method" refers to a method for averaging a predetermined value based on a regression function. Two examples of the regression characteristic value average method are described herein. One is a normal regression characteristic value average method, and another is a regression characteristic value average method using average information content. Fig. 3A shows the procedural flow of a normal regression characteristic value average method. Fig. 3B shows the procedural flow of a regression characteristic value average method using average information content.

1-1. Normal Regression Characteristic Value Average Method (Fig. 3A)

First, regression analysis for the plot "Cumulative consumption quantity (yi) - Elapsed time (x)" is conducted for the nutrient sources. Herein, "I (=1, 2, ..., n)" shows identification numbers for the nutrient sources, and "n," representing the number of the nutrient sources to be used, is the maximum number (and such numbers are used hereinafter). Therefore, the "n" regression function is acquired through the corresponding treatment.

Subsequently, a characteristic value (ai) is acquired based on the obtained regression function (0302a). That is to say, characteristic value ($\alpha$i) is computed for each nutrient source through the corresponding treatment. The term "characteristic value ($\alpha$i)" refers to estimates of the cumulative consumption quantity acquired through a regression function at a specific time, estimates of the rate of consumption acquired through slopes of the regression function at a specific time, and the time average of estimates of the cumulative consumption quantity resulting when integral values of the regression function from the commencement of culture until a specific time are divided by the elapsed time (hereinafter simply referred to as "Time Average of Estimates of the Cumulative Consumption Quality"). Herein, the "specific time" is not particularly limited. However, use of the time at which difference in the rate of consumption by the microbial species is the most remarkable is desirable.

Next, a characteristic value ($\alpha$i) computed based on the nutrient sources is averaged for all of "n" nutrient sources, and the average characteristic value (A) is acquired (0303a). That is to say, an average characteristic value (A) for a soil to be analyzed through the corresponding treatment is acquired.

It can be said that the larger the average characteristic value (A) becomes, the better the corresponding soil is suitable for a crop cultivation in which microorganisms are highly activated. This is because a plurality of the nutrient sources whose rate of consumption differs depending on microbial species are used in the present invention. Thus, only partial nutrient sources are consumed in soil with fewer types of microorganisms. As a result, the characteristic value remains low on average. On the other hand, all nutrient sources are evenly consumed in soil with diverse types of microorganisms. As a result, average characteristic value becomes high.

Furthermore, the corresponding average characteristic value (A) is used for conversion of soil to be analyzed into crop cultivation frequency (0304a). In regards to the method of computation of the corresponding conversion, a possible method for an inferior-to-superior comparison of soil to be analyzed and benchmark soil may be acceptable in terms of the results of computation for crop cultivation frequency. For instance, the characteristic value intervals are separated based on the comparison with benchmark soil regarding which crop cultivation effect is clear, and conversion into a step-by-step ranking, such as A, B, and C, etc., may be acceptable. Additionally, if the corresponding comparison is possible, characteristic values themselves may simply be used. In such case, characteristic values themselves represent the crop cultivation frequency.

Therefore, the crop cultivation frequency of relevant soil is compared with that of benchmark soil. If the crop cultivation frequency of relevant soil is greater than that of the benchmark soil, such soil is considered to be more suitable for crop cultivation. On the other hand, If the crop cultivation frequency of relevant soil is less than that of the benchmark soil, such soil is considered to not to be suitable for crop cultivation. In addition, in case that benchmark soil is not established, if comparison takes place among 2 or more soils to be analyzed, the values for crop cultivation frequency of different soils to be analyzed may simply be compared.

In addition, the type of the regression function acquired through the aforementioned regression analysis treatment (0301 a) is not particularly limited. However, a logistic curve and other growth curves are applied. More preferably, a sigmoid curve based on a Gompertz function formula is used. Additionally, linear regression may be used. Although the accuracy of a linear model is low, such model is effective for maintaining low computation costs. Although a non-linear model is effective in that the accuracy of such model is high, the computation costs will accordingly be high. Moreover, the average achieved through the aforementioned average treatment (0303a) is not particularly limited to arithmetic mean, geometric mean, harmonic mean, and the like. The same applies to the averages stated hereinafter.

1-2. Regression Characteristic Value Average Method Using Average Information Content (Fig. 3B)

First, regression analysis concerning the plot "Cumulative consumption quantity (yi) - Elapsed time (x)" is conducted for the nutrient sources (0301 b). Next, characteristic values ($\alpha$i) are acquired based on the obtained regression function (0302b). Characteristic values ($\alpha$i) computed are averaged for all nutrient sources, and the average characteristic value (A) is acquired (0303b). The steps used are the same as those described in relation to Fig. 3 above (0301a, 0302a, and 0303a).

Subsequently, the average information content (E) for the characteristic values ($\alpha i$) computed based on the nutrient sources is computed via step 0302b (0304b). That is to say, bias of the cumulative consumption quantity of the nutrient sources is acquired using the concept of the average information content. In addition, the order for performance of either the treatment of step 0303b or that of step 0304b does not matter. Moreover, the method for computation of the average information content (E) is stated later.

Next, the average information content (E) is multiplied by the average characteristic value (A) computed through step 0303b. Herein, the greater the bias in regards to the nutrient sources of the cumulative consumption quantity becomes, the lower the average information content (E) becomes. The lower the bias becomes, the greater the value of average information content (E) becomes. Additionally, the term "bias of the cumulative consumption quantity" refers to a bias of assimilation regarding the nutrient sources. Thus, the corresponding average information content (E) can be said to be the value in which the degree of diversification of living microorganisms is reflected. Therefore, the greater the degree of diversification of the living microorganisms is, the greater the value of the corresponding average information content (E) is. The lower the degree of diversification thereof is, the lower the value of the corresponding average information content (E) is. Thus, the corresponding multiplication value (AE) can be used for measurement of the degree of crop cultivation of soil to be analyzed as an indicator in which the degree of diversification of the living microorganisms is reflected. Thus, in the same manner as with the method described in Section 1-1, the corresponding multiplication value (AE) is converted in a manner that is comparable with the manner used for benchmark soil or other soils to be analyzed, or the corresponding multiplication value is used. Thereby, such value represents the crop cultivation frequency of the soils to be analyzed, and is the measurement result thereof (0306b).

In addition, the average information content E is given by the following formula in relation to the probability "pi" of occurrence of the event "i."

$$E = -\textstyle\sum p_i \log p_i \text{ (however; } \textstyle\sum \text{ will be applied for i.)}$$

Now, in regards to the characteristic value "$\alpha i$" that corresponds to the nutrient sources "i," if the percentage of the total for all nutrient sources is "pi," the following formula applies.

$$p_i = \alpha_i / \textstyle\sum \alpha_j \text{ (however; } \textstyle\sum \text{ will be applied for j.)}$$

When "p" for all nutrient sources is 1/n (where "n" represents the number of the nutrient sources), if the maximum value is obtained and the number that is the base of a logarithm is "n," the average information content will be 1.0.

At such time, the following formula applies.

$$E = -\textstyle\sum (\alpha_i / \textstyle\sum \alpha_j)\, \log_n p_i = \textstyle\sum \alpha_j\, \log_n p_i / \textstyle\sum \alpha_j$$

At the same time, arithmetic mean (A) is defined as follows.

$$A = \textstyle\sum \alpha_j / n$$

Thus, when E is multiplied by the arithmetic mean, the following formula applies.

$$AE = -\textstyle\sum \alpha_i\, \log_n p_i / n$$

That is to say, compared with the simple average, since only the expression "$\log_n p_i$" is added, it is possible to consider a kind of weighted average.

2. Average Data Regression Method

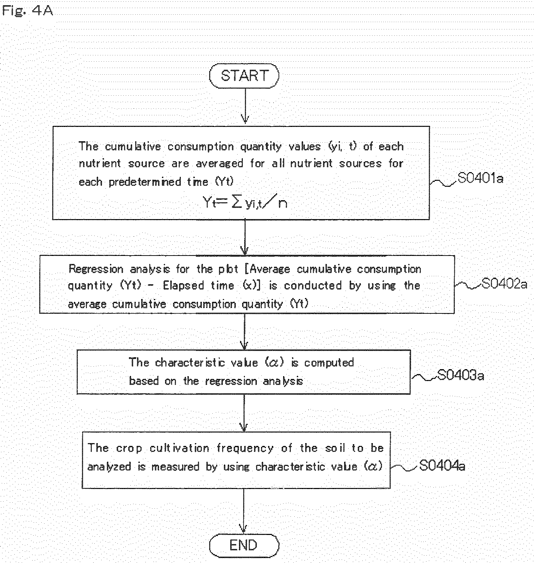

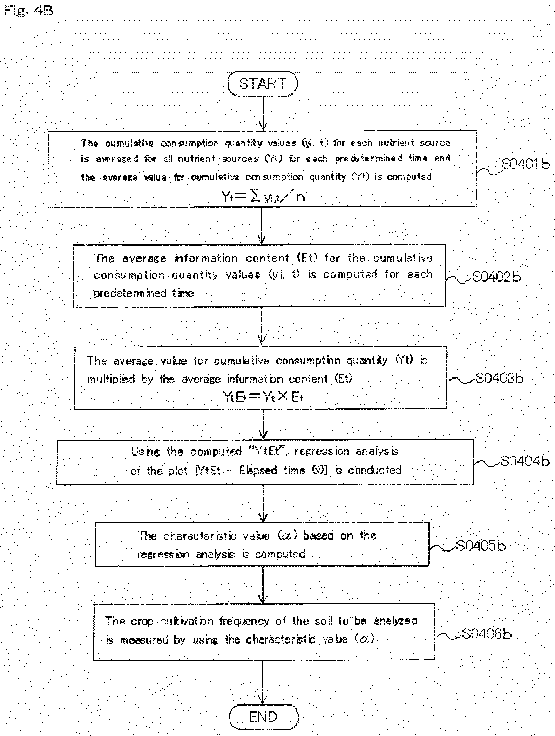

The average data regression method is described as follows. That is to say, following averaging of measurement results for the cumulative consumption quantity at each time for all nutrient sources, regression analysis is conducted under the condition that time is an explanatory variable and the corresponding average value for each time is a dependent variable. Two methods are explained hereinafter. One of them is a normal average data regression method, and another is an average data regression method that uses average information content. Fig. 4A shows the procedural flow for the former, and Fig. 4B shows the procedural flow for the latter.

2-1. Average Data Regression Method (Fig. 4A)

First, the cumulative consumption quantity values (yi, t) of each nutrient source will be averaged for all nutrient sources (Yt) (0401 a) for each predetermined time. That is to say, based on the plot [Cumulative consumption quantity (yi, t) - Elapsed time (x)] acquired for each source of nutrient through the corresponding treatment, the plot [Cumulative consumption quantity (Yt) - Elapsed time (x)] will be obtained. The crop cultivation frequency of the soil to be analyzed is measured by using the corresponding average single plot [Cumulative consumption quantity (Yt) - Elapsed time (x)].

Subsequently, regression analysis for the corresponding average single plot [Cumulative consumption quantity (Yt) - Elapsed time (x)] will be conducted (0402a). The regression analysis is subject to "1. Regression Characteristic Value Average Method."

Next, the characteristic value ($\alpha$) based on the regression analysis will be computed (0403a). The corresponding characteristic value ($\alpha$) is subject to "1. Regression Characteristic Value Average Method." The conversion of the corresponding characteristic value ($\alpha$) takes place in a manner that is comparable with the manner used for benchmark soil or other soils to be analyzed, or the corresponding characteristic value ($\alpha$) is used as it is. Thereby, such resultant value can be used for crop cultivation frequency and corresponds to the results of measurement of the crop cultivation frequency of the soil to be analyzed (0404a).

2-2. Average Data Regression Method Using Average Information Content (Fig. 4B)

First, the cumulative consumption quantity values (yi, t) for each nutrient source is averaged for all nutrient sources (Yt)(0401 b) for each predetermined time. Next, the average information content (Et) for the cumulative consumption quantity values (yi, t) will be computed (0402b) for each predetermined time. The order of preference for the treatment in step 0401 b and step 0402b does not matter. The average value for cumulative consumption quantity (Yt) is multiplied by the average information content (Et), and a multiplication value is obtained (YtEt) in which bias of the cumulative consumption quantity of each nutrient source is reflected (0403b).

Subsequently, using the corresponding multiplication value (YtEt), regression analysis of the plot [YtEt - Elapsed time (x)] is conducted (0404b), and the characteristic value ($\alpha$) based on the regression analysis is computed (0405b). The corresponding characteristic value ($\alpha$) is subject to "1. Regression Characteristic Value Average Method." The conversion of the corresponding characteristic value ($\alpha$) takes place in a manner that is comparable with the manner used for benchmark soil or other soils to be analyzed, or the corresponding characteristic value ($\alpha$) is used as it is. Thereby, such resultant value can be used as the crop cultivation frequency and corresponds to the results of measurement of the crop cultivation frequency of the soil to be analyzed (0406b).

3. Simplified Method Not Using Regression Analysis

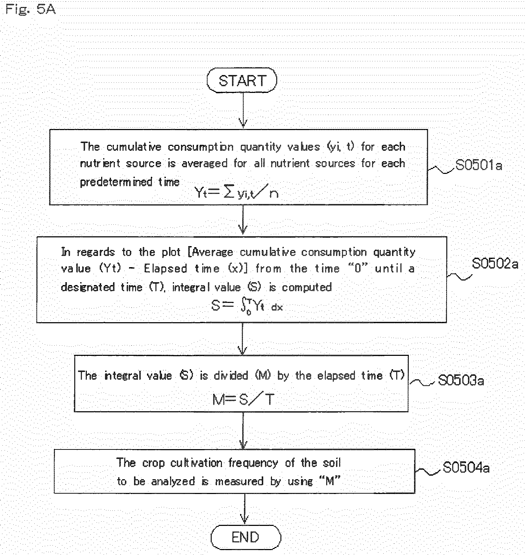

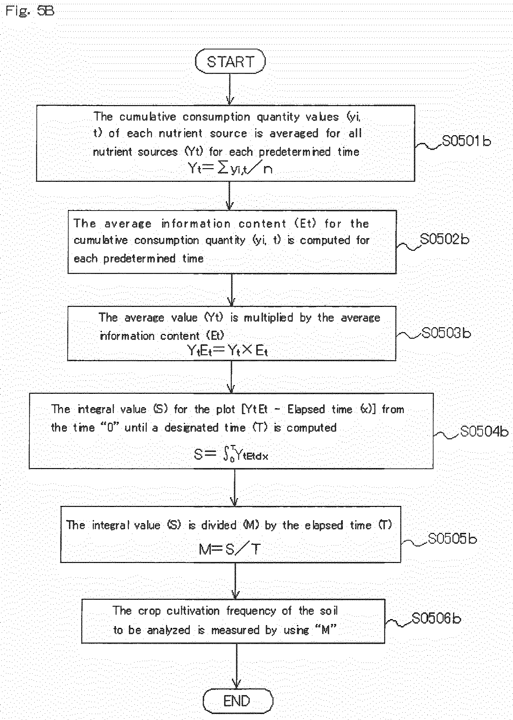

Two examples of a more simplified method not using regression analysis are explained hereinafter. One is a simplified method not using the average information content, and the other is a simplified method using the average information content. Fig.5A is a procedural flow chart for the former method, while Fig.5B is a procedural flow chart for the latter method.

3-1. Simplified Method Not Using Average Information Content (Fig. 5A)

First, the average value of the cumulative consumption quantity values (yi, t) for each nutrient source (Yt) is computed for all nutrient sources for each predetermined time (0501 a). That is to say, based on the corresponding treatment, average single plot [Average cumulative consumption quantity value (Yt) - Elapsed time (x)] can be obtained through the plot [Cumulative consumption quantity value (yi, t) - Elapsed time (x)] for all nutrient sources. Next, in regards to the corresponding plot [Average cumulative consumption quantity value (Yt) - Elapsed time (x)], integral value (S) from the time "0" until a designated time (T) is computed (0502a). Next, the integral value (S) is divided by the elapsed time (T) (0503a). The corresponding division value (M) is converted in a manner that is comparable with the manner used for benchmark soil or other soils to be analyzed, or the corresponding division value (M) is used as it is. Thereby, such resultant value can be used for crop cultivation frequency and corresponds to the results of measurement of the crop cultivation frequency of the soil to be analyzed (0504a). In addition, in case that the cumulative consumption quantity is measured at equal intervals, the method used will be the logically equivalent to a method for regression corresponding to the linear function [y=b (b=constant)] in "2-1. Average Data Regression Method" mentioned above.

3-2. Simplified Method Using Average Information Content (Fig. 5B)

First of all, the average value for the cumulative consumption quantity values (yi, t) of each nutrient source will be computed for all nutrient sources (Yt) for each predetermined time (0501 b). The average information content (Et) for the cumulative consumption quantity (yi, t) is computed for each predetermined time (0502b).The average value of the cumulative consumption quantity (Yt) computed through step 0501 b is multiplied by such average information content (Et). A multiplication value (YtEt) that reflects bias of the cumulative consumption quantity of each nutrient source is obtained (0503b). The Integral value (S) for the corresponding plot [YtEt - Elapsed time (x)] is computed (0504b). The corresponding integral value (S) is divided by the elapsed time (T) (0505b). And the corresponding division value (M) is converted is converted in a manner that is comparable with the manner used for benchmark soil or other soils to be analyzed, or the corresponding division value (M) is used as it is. Thereby, such resultant value can be used as the crop cultivation frequency and corresponds to the results of measurement of the crop cultivation frequency of the soil to be analyzed (0506b). in addition, in case that the cumulative consumption quantity is measured at equal intervals, the method used will be the logically equivalent to a method for regression corresponding to the linear function [y=b (b=constant)] in "2-2. Average Data Regression Method Using Average Information Content" mentioned above.

<Example 1>

Specific examples in which the method for measuring crop cultivation frequency of the first embodiment targets actual soil are described hereinafter.

The crop cultivation frequency of the soil to be analyzed was measured using the OmniLog Identification System (a microorganism identification system) of Biolog Inc. For the present example, the cumulative consumption quantity was indirectly observed using the degree of color of redox reagents described below.

1. Creation of Microorganism Samples

Samples of soil to be analyzed were collected. The soil used did not pass through a 4 mm sifter. Soil containing high moisture content may be used after such soil has been slightly dried in the shade. Additionally, depending on soil granularity, a 2-mm sifter may be used, or moist field soil may be used as it is. In addition, in the this Example, four samples of soil to be analyzed were collected. Such soil samples were [1071], [1157], [1433], and [321], respectively.

10 g of each of sifted soil sample was removed, and such soil was diluted 10-fold with 90 ml phosphate buffer solution (sterile) (20 mmol). Such soil was shaken for 12 hours to 16 hours at 150 -180 rpm. After shaking, such soil remained static for 3 minutes or longer, 1 ml thereof was obtained from the center thereof, the numbered soil samples were diluted with 99 ml sterile water, and a 1,000-fold diluted solution was produced.

2. OmniLog Identification System Setting

The 150 $\mu$l microorganism samples mentioned above was dispensed into separate wells of the GN2 microplate of the OmniLog Identification System. Using 1250 $\mu$l $\times 8$ channel pipettes for dispensing, the microorganism samples were removed into sterile reservoirs and dispensing took place. Herein, the microplate contains 96 wells ($8 \times 12$ wells arranged in vertical and horizontal rows), and each well was filled beforehand with water and one of 95 types of carbon sources (Table 1). Additionally, each well was filled with a redox reactive reagent (2,5 - diphenyl - 3 - (1 - naphthyl) - 2H - tetrazole - 3 - ium3 - (1 - naphthyl) - 2, 5 - diphenyl - 2H - tetrazole - 3 - ium) in a dry condition.

[Table 1] Type of Carbon Sources

Next, a GN2 microplate into which the microorganism samples had been dispensed was placed in an incubator of the OmniLog Identification System, and such microplate was cultured at 25°C for 48 hours using the OL_PM system. Herein, the degree of coloration for each well in the GN2 microplate was noted at 15-minute intervals over 48 hours following the commencement of culture.

3. Results

At 15-miniute intervals until 47.25 hours immediately following commencement of culture (0.00 hours) of soil numbers [1071], [1157], [1433], and [321], color results of each well were read, and crop cultivation frequency was determined using such color result data.

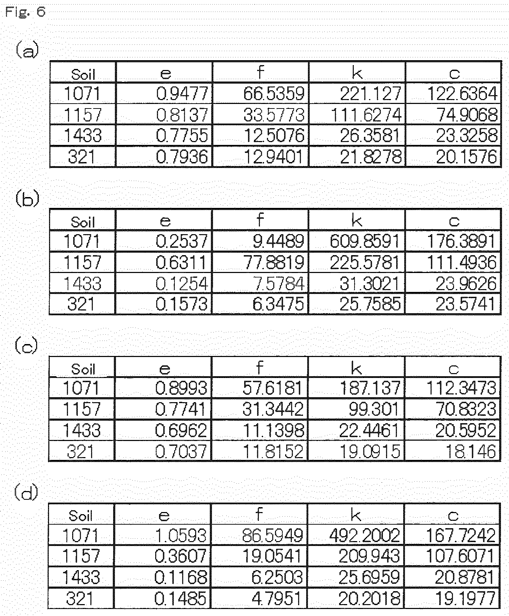

Results based on the aforementioned program for 4 soil samples with soil numbers [1071], [1157], [1433], and [321] are shown in Fig. 6 through Fig.9.

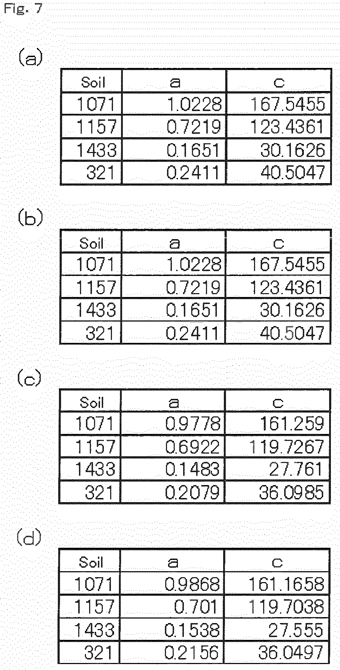

Fig. 6 shows the results from a case using a Gompertz curve as a regression function. (a) shows results from a normal regression characteristic value average method, (b) shows results from a regression characteristic value average method using average information content, (c) shows results from an regression characteristic value average method using average information content, and (d) shows results from an average data regression method using average information content. The aforementioned information is the same for Fig. 7 through Fig. 9 below. Moreover, "e" represents the slope at an inflection point as stated above, "f" represents cumulative consumption quantity, and "k" represents cumulative consumption quantity at infinite time. Furthermore, "c" represents cumulative consumption quantity following the elapse of 47.25 hours.

In Fig. 6, the greater the values for "e," "f," "k," and "c" become, the greater the crop cultivation frequency is. That is to say, it can be said that the corresponding soils are more suitable for crop cultivation. In Fig. 6 (a) through (d), all values for "e," "f," "k," and "c" in [1071] and [1157] are larger than those in [1433] and [321]. Therefore, when the corresponding 4 soil samples were compared, [1071] and [1157] showed results for good soil with relatively high crop cultivation frequency, and [1433] and [321] showed results for poor soil with relatively low crop cultivation frequency.

Fig. 7 shows the results for a case in which "y = ax + b" was used as a regression function. "a" represents slope and "c" represents the cumulative consumption quantity following elapse of 47.25 hours. In Fig. 7, the larger the values of "a" and "c" become, the larger the crop cultivation frequency is. That is to say, it can be said that the corresponding soil is more suitable for crop cultivation. Therefore, in case that the corresponding 4 soil samples were compared, [1071] and [1157] showed results for good soil with relatively high crop cultivation frequency, and [1433] and [321] showed results for poor soil with relatively low crop cultivation frequency. These were the same results as those for Fig. 6 stated above.

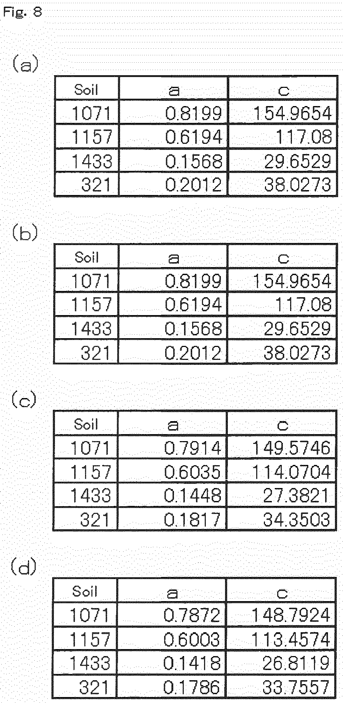

Fig. 8 shows the results for a case in which "y = ax" was used as a regression function. "a" represents the slope. In Fig. 8, the larger the values of "a" become, the larger the crop cultivation frequency is. That is to say, it can be said that the corresponding soil is more suitable for crop cultivation. Therefore, when the corresponding 4 soil samples were compared, [1071] and [1157] showed results for good soil with relatively high crop cultivation frequency, and [1433] and [321] showed results for poor soil with relatively low crop cultivation frequency. These were the same results as those for Fig. 6 and Fig. 7 stated above.

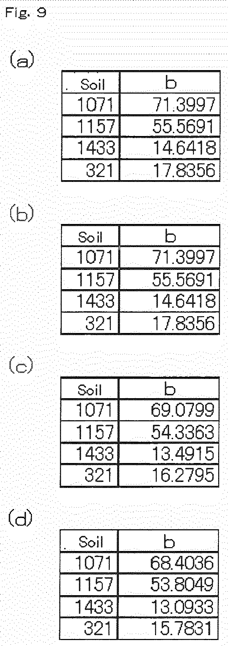

Fig. 9 shows the results for a case in which "y = b" was used as a regression function. "b" shows cumulative consumption quantity. In Fig. 9, the larger the values of "c" become, the larger the crop cultivation frequency is. That is to say, it can be said that the corresponding soil is more suitable for crop cultivation. Therefore, when the corresponding 4 soil samples were compared, [1071] and [1157] showed results for good soil with relatively high crop cultivation frequency, and [1433] and [321] showed results for poor soil with relatively low crop cultivation frequency. These were the same results as those for Fig. 6 through Fig. 8 stated above.

<Example 2>

<Outline>

Example 2 is related to the second aspect of the present invention. In the second aspect of the present invention, the method for measuring crop cultivation frequency of the first aspect of the present invention is applied in order to determine whether deception has taken place regarding the production region of an agricultural product.

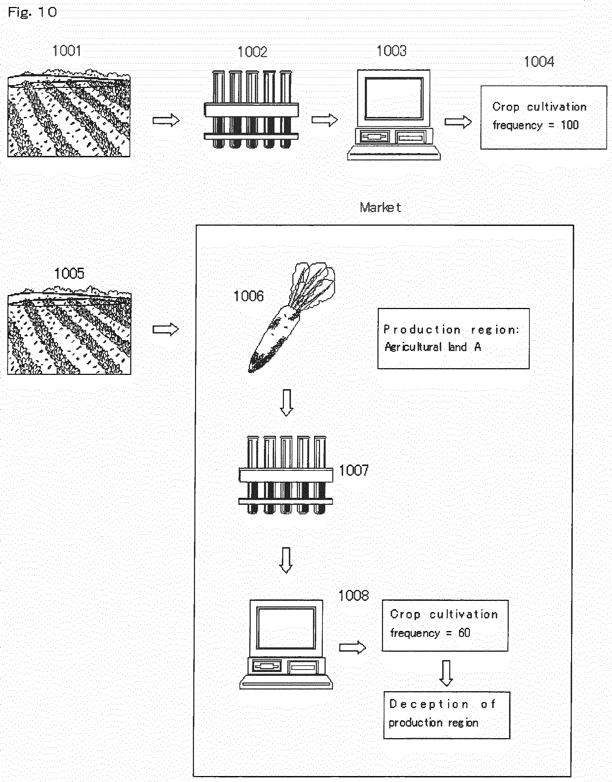

Fig. 10 shows an outline of the method for determining the authenticity of the production region of an agricultural product of Example 2. First, the crop cultivation frequency of soil of specific prime agricultural land A (1001), which was a target of deception regarding the production region of an agricultural product, was determined. Specifically, such measurement was conducted based on the following procedures. First, soil from the aforementioned prime agricultural land A was collected as benchmark soil. In the same method as that used for Example 1, a suspension of soil was added dropwise to a plurality of nutrient sources, and the cumulative consumption quantity of the nutrient sources was observed (1002). Next, in regards to time-series data for the obtained cumulative consumption quantity of the nutrient sources, calculation was conducted using regression analysis or the like, in the same manner as with Example 1 (1003), and the crop cultivation frequency of prime agricultural land A mentioned above was acquired (1004).

Secondly, crop cultivation frequency was measured for soil attached to agricultural products that were targets of judgment as to whether or not deception had taken place regarding the corresponding production region. Specifically, such measurement was conducted based on the following procedures. Agricultural products (1006) were harvested from poor agricultural land B (1005) that differed from the prime agricultural land A mentioned above. However, in order to sell the corresponding agricultural products at a higher price than the actual market price, agricultural products fraudulently labeled as coming from prime agricultural land A mentioned above as the production region were brought to market. In order to bring such products to market, it was necessary for farmers to ship agricultural products to which soil was attached in advance. Soil from the agricultural land B was attached to the aforementioned agricultural products with fraudulent labeling. Concerned parties with a relationship to the market collected the soil attached to the agricultural products mentioned above. Via the same method as that used when measuring the crop cultivation frequency of the prime agricultural land A mentioned above, the cumulative quantity of the nutrient sources consumed by the microorganisms contained in such soil was observed (1007).Using the acquired data, calculation was conducted using the same method as that used when measuring the crop cultivation frequency of the prime agricultural land A mentioned above, and the crop cultivation frequency of the soil attached to the aforementioned agricultural land was measured (1008).

Lastly, the crop cultivation frequency of the prime agricultural land mentioned above and that for the soil attached to the agricultural products mentioned above were compared. If a relevant value differed beyond a predetermined value, deception regarding the production region of such agricultural products was considered to have taken place.

<Configuration>

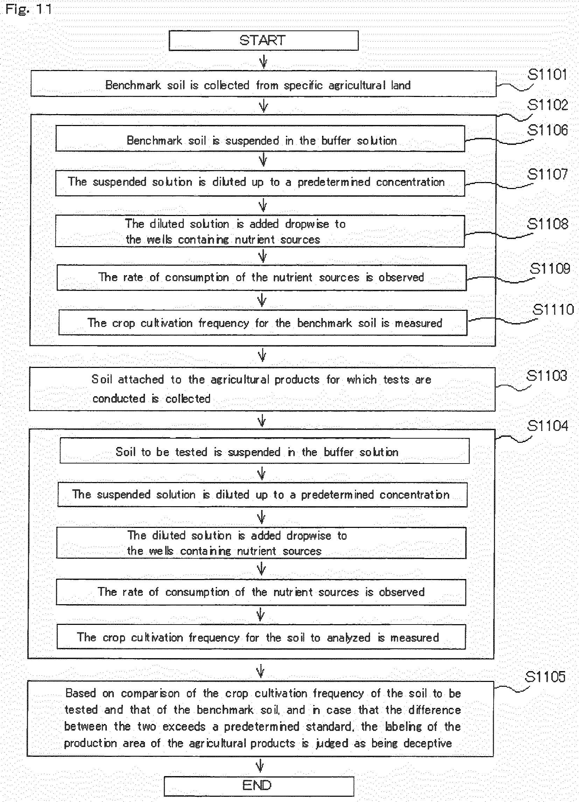

Fig.11 is a flow chart of a method for assessing production region deception related to the second embodiment. The method for assessing production region deception related to such embodiment comprises a first step of collecting benchmark soil, a second step of measuring the crop cultivation frequency of the benchmark soil, a third step of collecting soil to be tested, a fourth step of measuring of the crop cultivation frequency of the soil to be tested, and a final step of determination regarding deception. Each step is explained in detail hereinafter.

The "first step of collecting benchmark soil" (1101) is a step of "collecting benchmark soil from specific agricultural land." The term "specific agricultural land" refers to land for agriculture within a region fraudulently designated a "production region" in the labeling of agricultural products. The term "benchmark soil" refers to soil collected from the aforementioned specific agricultural land that represents the aforementioned specific agricultural land and constitutes a standard. Such soil may be collected from a single location or a plurality of locations on the aforementioned specific agricultural land.

In the "second step of measuring the crop cultivation frequency of the benchmark soil" (1102), "the suspension of the benchmark soil is added to a plurality of nutrient sources, the rates of consumption of least some of which differ depending upon microbial species, the cumulative consumption quantity of the nutrient sources consumed by the microorganisms contained in the benchmark soil within a certain period is observed, and in which crop cultivation frequency of the aforementioned benchmark soil is measured" The benchmark soil collected in the first step of collecting of benchmark soil was suspended in the buffer solution (1106), the suspended solution was diluted up to a predetermined concentration (1107), such diluted solution was added dropwise to the wells containing nutrient sources (1108), and the rate of consumption of the nutrient sources was observed

(1109). The crop cultivation frequency is measured based on the acquired rate of consumption of the nutrient sources. Such procedures are the same as those mentioned for Example 1, and thus the details thereof are omitted.

The "third step of collecting the soil to be tested" (1103) is a step in which "soil attached to the agricultural products in which the specific agricultural land is labeled as a production region is collected as soil to be tested." The expression "the agricultural products in which the specific agricultural land is labeled as a production region " refers to agricultural products subject to a test for evaluation of deception of production region. The term "soil to be tested" refers to the soil attached to the aforementioned agricultural products, the crop cultivation frequency of which should be compared with that of the benchmark soil mentioned above. From among the agricultural products shipped to market, soil may be collected arbitrarily from a single or a plurality of agricultural product(s).

The "fourth step of measuring the crop cultivation frequency of the soil to be tested" (1104) is a step in which "the suspension of the soil to be tested is added dropwise to the same types of nutrient sources as those used in the second step of measuring of the crop cultivation frequency of the benchmark soil, in which the cumulative consumption quantity of the nutrient sources consumed by the microorganisms contained in the soil to be tested within a certain period is observed, and in which the crop cultivation frequency of the soil to be tested is measured." The same procedures as those used in the second step of measuring crop cultivation frequency of the benchmark soil were used, targeting the soil to be tested. Care should be taken to use the same types of nutrient sources as those used in the second step of measuring the crop cultivation frequency of the benchmark soil and to observe the cumulative consumption quantity under the same conditions and at the same points of time. This is because the soil to be tested and the benchmark soil should be compared under the same conditions.

The final step of determination regarding deception (1105) is "based on comparison of the crop cultivation frequency of the soil to be tested and that of the benchmark soil, and in case that the difference between the two exceeds a predetermined standard, the labeling of the production area of the agricultural products is judged as being deceptive." As stated in Example 1, the crop cultivation frequency could be indicated by numbers or scale phase evaluation, such as with A, B, C, D, and E. In case that the crop cultivation frequency is indicated by numbers, the "predetermined standard" is also a numeric value. When the "predetermined standard" becomes excessively high, cases in which it is possible to evade detection of deception of production region increase. If the "predetermined standard" becomes excessively low, cases in which deception of production region is erroneously determined to have taken place increase. Therefore, the "predetermined standard" is determined under the condition that several soils are tested and appropriate numeric values are selected for the results of such test that do not place disproportionate weight on either side. If the crop cultivation frequency is indicated by scale phase evaluation, the "predetermined standard" is the value related to a difference in phase.

As stated above, through comparing the soil attached to an agricultural product with soil from the production region indicated via labeling, the method for determining the authenticity of the production region of an agricultural product of the second aspect of the present invention enables detection of production region deception via a simplified method.

Example 3

<Outline>

Example 3 is related to the third aspect of the present invention. The method for determining the authenticity of the production region of an agricultural product of the second aspect of the present invention enables judgment only through comparison of the crop cultivation frequency as a sole value computed for each soil. Thus, such method entails simplification. Therefore, despite the convenience of such simplification, there is a limit to the accuracy thereof.

The first reason why the accuracy of judgment declines is stated as follows. That is to say, different soils have clearly unique characteristics regarding diversification of microorganisms. However, when such characteristics are indicated by a single numeric value for crop cultivation frequency, such value is remarkably effective for the purpose of determining of suitability of crop cultivation regarding relevant soils. However, crop cultivation frequency is not always accurate for the purpose of determining the identity of soil, which has been problematic. That is to say, two soils with clearly differing levels of diversification of microorganisms may be nearly equal in suitability in terms of crop cultivation. Therefore, the values for crop cultivation frequencies are approximately equal, and such two soils are judged as being the same. In other words, according to the method for determining the authenticity of the production region of an agricultural product of the second aspect of the present invention, agricultural products that have been produced in poor production regions in which suitability of crop cultivation is lower than that stated on labeling can be judged as having being produced in mislabeled production regions. However, agricultural products produced in mislabeled production regions that are equal in terms of fertility to labeled production regions could not be accurately evaluated, which has been problematic.

The second reason for the lack of accuracy of judgment is that there is problematic moderate variation of dilution concentration among a plurality of soils when producing a suspension containing microorganisms from such soils. That is to say, a suspension may be diluted to a greater extent than necessary for tests of agricultural products the production region of which is properly labeled. As a result, the production region of authentic agricultural products has been erroneously evaluated as being mislabeled. However, there has been fear that insufficient dilution of suspension for the tests could result in evasion of tests for agricultural products with mislabeled production regions.

Based on the method for determining the authenticity of the production region of an agricultural product of the third aspect of the present invention, the following problems were solved via the following methods. First, in regards to the first reason for the lack of accuracy of judgment, crop cultivation frequency as the sole numeric value computed for each soil should not be compared. Instead, in order to enhance the accuracy of judgment, comparison at the level of the nutrient sources was made. More specifically, microorganism types are remarkably diverse, and the percentage of inhabitation varies widely depending on each soil. No two people that have the same fingerprint exist in the world. In the same manner, there exist no soils that are absolutely the same. When diversification of microorganisms in the soil is measured based on a large number of nutrient sources that are consumed at different rates by different microbial species is measured, what and to what extent nutrient sources are consumed can cause different results for different soils. Thus, the identity of soil can be judged.

Next, the second problem was solved as follows. That is to say, even if the concentrations of a diluted suspension are different for different soils, the percentages representing the number of the diverse living microorganisms contained in such suspension do not vary. Therefore, even differences in concentrations of suspensions have no effect on percentages of nutrient sources consumed. Without comparing absolute values for cumulative consumption quantity, the problem was solved by comparing the benchmark soil and the soil to be tested in terms of consumption percentages for nutrient sources.

<Configuration>

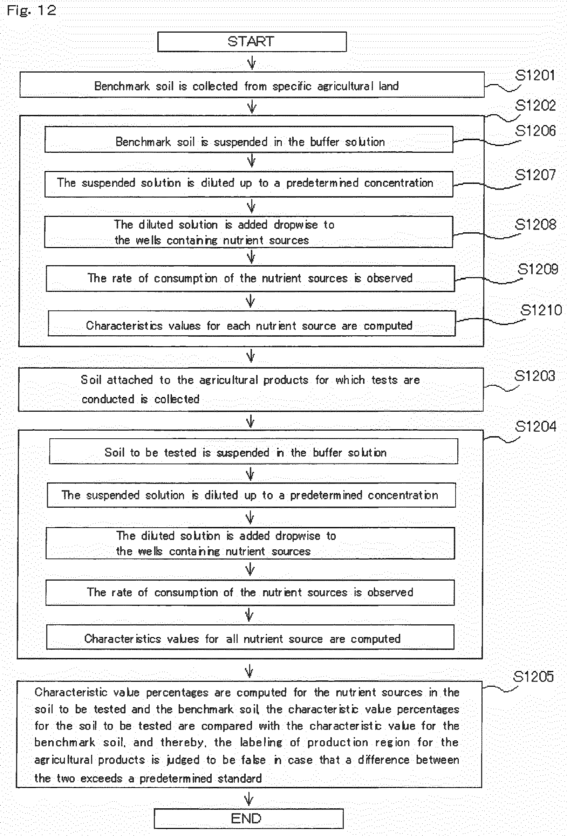

Fig.12 is a flow chart of a method for assessing production region deception related to the third embodiment. The method for assessing production region deception related to such embodiment comprises a first step of collecting the benchmark soil, a second step of computing characteristic value of the benchmark soil, a third step of collecting the soil to be tested, a fourth step of computing the characteristic value of the soil to be tested, and a final step of judgment.

Each step is explained in detail hereinafter.

The "first step of collecting of the benchmark soil" (1201) is the same as that of Example 2.

In the "second step of computing characteristic value of the benchmark soil" (1202) the benchmark soil is suspended in the buffer solution (1206), the suspended solution is diluted to a predetermined concentration (1207), the diluted solution is added dropwise into the wells containing nutrient sources (1208), and the rate of consumption of the nutrient sources is observed (1209). The step thus far is the same as that of Example 2. What is finally obtained is not the crop cultivation frequency but rather characteristics values for each nutrient source before crop cultivation frequency is determined (1210). This point is different from Example 2. Characteristic values were explained in detail in Example 1.To repeat, characteristic values correspond to estimates of cumulative consumption quantity acquired through the regression function at a specific time, estimates of the rate of consumption acquired through slopes of the regression function at a specific time, and a time average of estimates of the cumulative consumption quantity resulting when the integral values for the regression function from the time following commencement of culture until a specific time are divided by elapsed time. The basic concept of computation of characteristic values is the same as that of Example 1. It should be noted that the purpose of Example 3 is to compute characteristic values for each nutrient source. Thus, there are no particular differences among the regression characteristic value average method, the average data regression method, or the simplified method as described in Example 1. Computation may be performed based on either regression analysis of the nutrient sources or simply by dividing the cumulative consumption quantity of the nutrient sources by the required time.

The "third step of collecting the soil to be tested" (1203) is the same step as that of Example 2.

The "fourth step of computing the characteristic value of the soil to be tested" (1204) differs from that of Example 2 in that characteristic values for each nutrient source are computed in lieu of crop cultivation frequency. Except for this point, other matters are the same. The same information stated in the second step of computing the characteristic value of the benchmark soil mentioned above is applied to the computation method for characteristic values.

In the "final step of determination regarding deception" (1205), "characteristic value percentages are computed for the nutrient sources in the soil to be tested and the benchmark soil, the characteristic value percentages for the soil to be tested are compared with the characteristic value for the benchmark soil, and thereby, the labeling of production region for the agricultural products is judged to be false in case that a difference between the two exceeds a predetermined standard."

The term "characteristic value percentage" refers to the "the characteristic value percentage for each nutrient source." For instance, nutrient sources A, B, C, and D have characteristic values of 100, 90, 70, and 60, respectively. As percentage is an issue, it is possible to use different notations depending upon the method of selection of the standard numeric value. Hypothetically, if A as one of the nutrient sources is designated as a standard, the ratio is (1.0 : 0.9 : 0.7 : 0.6). Alternatively, if the value 80 as an average of four characteristic values is designated, the ratio is (1.250 : 1,125 : 0.875 : 0.750).

The characteristic value percentage for the benchmark soil is acquired based on the characteristic values for each nutrient source of the benchmark soil acquired through the aforementioned second step of computing of characteristic value of the benchmark soil, and the characteristic value percentage is acquired for the soil to be tested based on the characteristic values for each nutrient source related to the soil to be tested acquired through the aforementioned fourth step of computing of characteristic value of the soil to be tested. Thereafter, the aforementioned percentages are compared. In case that the difference between the two exceeds a predetermined standard, the labeling of the production area of the aforementioned agricultural products is judged as being deceptive. A specific example of a method for comparison is stated as follows.

For instance, in the same manner as described in the preceding paragraph, nutrient sources A, B, C, and D exist, and the characteristic values thereof are (100, 90, 70, and 60) for the benchmark soil and (80, 72, 56, and 48) for the soil to be tested. In each case, the average values of the characteristics are 80 and 64. If the characteristic value percentages therefor are computed, the results in both cases are (1.250 : 1,125 : 0.875 : 0.750). The two match, and the difference between the two cases is zero. Thus, it is judged that no mislabeling took place concerning the production region.

However, in reality, the characteristic value ratio is not completely matched from a practical standpoint as stated in the case of the preceding paragraph. Therefore, it is necessary to define the "difference between the two" and "predetermined standard." Such terms can be defined in various manners for the purpose of determining deception of production regions with a certain level of accuracy. Specific examples are provided as follows.

In the same manner mentioned above, nutrient sources A, B, C, and D exist, and the characteristic values thereof are (100, 90, 70, and 60) for the benchmark soil and (85, 70, 59, and 46) for the soil to be tested. Given this, the characteristic value percentages represented by the percentages to average characteristic values are (1.250 : 1,125 : 0.875 : 0.750) and (1.308 : 1.077 : 0.908 : 0.708). The differences between the two are ("0.058, - 0.048, 0.033, - 0.042). As methods for definition related to the "difference between the two," a method in which 0.081 is the total absolute values of the aforementioned values or a method in which sum of squares of the aforementioned values is 0.0085 may be used.

Furthermore, the characteristic value percentages are acquired for various soils, and based on such resultants, standard deviation is obtained for the numeric values for the percentage of each nutrient source. By dividing the difference mentioned above (0.058, - 0.048, 0.033, - 0.042) by the aforementioned deviation, the sum or sum of squares of the absolute values thereof may be used. Moreover, upon computing the sum in total, weighing of the numeric values of the nutrient sources through computation of the average information content as introduced in Example 1 may be carried out.

In regards to the "difference between the two" defined as above, "predetermined standard" may be determined. More specifically, judgment takes place using various soils, and a certain degree of accuracy thereof is selected. When the predetermined standard is overestimated, the number of cases of test evasion increases. When the predetermined standard is underestimated, cases of erroneously evaluated deception increase. Therefore, appropriate values are selected using experimental data.

As described above, the agricultural products with deceptive production regions can be judged with high accuracy according to the method for determining the authenticity of the production region of an agricultural product of the third aspect of the present invention.

## Claims

1.   A method for judging the authenticity of the production region of an agricultural product, comprising:

a first step of collecting of the benchmark soil, in which the benchmark soil is collected from a specific agricultural land;
a second step of computing characteristic value for the benchmark soil, in which the suspension of benchmark soil is added dropwise to a plurality of nutrient sources, the rates of consumption of least some of which differ depending upon microbial species, in which quantity of the nutrient sources consumed by the microorganisms contained in the benchmark soil within a certain period is observed as cumulative consumption quantity of the benchmark soil, and in which characteristic values for the benchmark soil for all nutrient sources are computed;
a third step of collecting of the soil to be tested in which the soil attached to the agricultural products for which the specific agricultural land is labelled as a production region as the soil to be tested is collected;
a fourth step of computing characteristic value of the soil to be tested in which the suspension of the soil to be tested is added dropwise to the same types of nutrient sources as those used in the second step of computing characteristic value of the benchmark soil, in which quantity of the nutrient sources consumed by the microorganisms contained in the soil to be tested within a certain period is observed as cumulative consumption quantity of the soil to be tested, and in which characteristic values related to the soil to be tested for all nutrient sources are computed; and
a final step of determination regarding deception, in which characteristic value percentages are computed for the nutrient sources in the soil to be tested and the benchmark soil, each characteristic value percentage being represented by a ratio of the characteristic value for one of the nutrient sources to an average of the characteristic values for all nutrient sources, the characteristic value percentages for the soil to be tested are compared with the characteristic value percentages for the benchmark soil, and thereby, the labelling of production region for agricultural products is judged to be false in case that a difference between the two exceeds a predetermined standard.

## Patentansprüche

1.   Verfahren zum Beurteilen der Authentizität der Erzeugungsregion eines landwirtschaftlichen Produkts, umfassend:

einen ersten Schritt zum Sammeln des Referenzbodens, in dem der Referenzboden von einem bestimmten landwirtschaftlichen Acker gesammelt wird;
einen zweiten Schritt zum Berechnen eines charakteristischen Wertes für den Referenzboden, in dem die Aufschwemmung von Referenzboden tropfenweise zu einer Mehrzahl von Nährstoffquellen, von welchen sich die Verbrauchsraten wenigstens einiger in Abhängigkeit von mikrobiellen Arten unterscheiden, hinzugefügt wird, in dem eine Quantität der durch die in dem Referenzboden enthaltenen Mikroorganismen verbrauchten Nährstoffquellen innerhalb einer bestimmten Periode als kumulierte Verbrauchsquantität des Referenzbodens aufgezeichnet wird und in dem charakteristische Werte für den Referenzboden für alle Nährstoffquellen berechnet werden;
einen dritten Schritt zum Sammeln des zu testenden Bodens, in dem der an den landwirtschaftlichen Produkten, für welche der bestimmte landwirtschaftliche Acker als eine Erzeugungsregion gekennzeichnet ist, anhaftende Boden als der zu testende Boden gesammelt wird;

einen vierten Schritt zum Berechnen eines charakteristischen Wertes des zu testenden Bodens, in dem die Aufschwemmung des zu testenden Bodens tropfenweise zu denselben Arten von Nährstoffquellen, wie jene in dem zweiten Schritt zum Berechnen eines charakteristischen Wertes des Referenzbodens verwendete, hinzugefügt wird, in dem eine Quantität der durch die in dem zu testenden Boden enthaltenen Mikroorganismen verbrauchten Nährstoffquellen innerhalb einer bestimmten Periode als kumulierte Verbrauchsquantität des zu testenden Bodens aufgezeichnet wird und in dem sich auf den zu testenden Boden beziehende charakteristische Werte für alle Nährstoffquellen berechnet werden; und

einen abschließenden Schritt zur Bestimmung betreffend Irreführung, in dem Prozentsätze charakteristischer Werte für die Nährstoffquellen in dem zu testenden Boden und dem Referenzboden berechnet werden, wobei jeder Prozentsatz charakteristischer Werte durch ein Verhältnis des charakteristischen Wertes für eine der Nährstoffquellen zu einem Durchschnitt der charakteristischen Werte für alle Nährstoffquellen repräsentiert ist, die Prozentsätze charakteristischer Werte für den zu testenden Boden mit den Prozentsätzen charakteristischer Werte für den Referenzboden verglichen werden und dabei die Kennzeichnung der Erzeugungsregion für landwirtschaftliche Produkte in dem Fall als fehlerhaft beurteilt wird, dass ein Unterscheid zwischen den beiden ein vorbestimmtes Maß übersteigt.

**Revendications**

1.  Procédé permettant de juger de l'authenticité de la région de production d'un produit agricole, comprenant :

une première étape consistant à recueillir le sol de référence, dans laquelle le sol de référence est recueilli à partir d'une terre agricole spécifique ;

une deuxième étape consistant à calculer une valeur caractéristique du sol de référence, dans laquelle la suspension du sol de référence est ajoutée goutte à goutte à une pluralité de sources d'éléments nutritifs, dont au moins certains des taux de consommation diffèrent en fonction des espèces microbiennes, dans laquelle la quantité des sources d'éléments nutritifs consommés par les microorganismes contenus dans le sol de référence dans un délai donné est observée comme une quantité de consommation cumulée du sol de référence, et dans laquelle des valeurs caractéristiques du sol de référence pour toutes les sources d'éléments nutritifs sont calculées ;

une troisième étape consistant à recueillir le sol à évaluer, dans laquelle le sol attaché aux produits agricoles, pour lesquels la terre agricole spécifique est labélisée en tant que région de production, en tant que sol à évaluer est recueilli ;

une quatrième étape consistant à calculer une valeur caractéristique du sol à évaluer, dans laquelle la suspension du sol à évaluer est ajoutée goutte à goutte aux mêmes types de sources d'éléments nutritifs que celles utilisées à la deuxième étape consistant à calculer une valeur caractéristique du sol de référence, dans laquelle la quantité des sources d'éléments nutritifs consommés par les microorganismes contenus dans le sol à évaluer dans un délai donné est observée comme une quantité de consommation cumulée du sol à évaluer, et dans laquelle des valeurs caractéristiques relatives au sol à évaluer pour toutes les sources d'éléments nutritifs sont calculées ; et

une étape finale consistant à déterminer une fraude, dans laquelle des pourcentages de valeur caractéristique sont calculés pour les sources d'éléments nutritifs dans le sol à évaluer et le sol de référence, chaque pourcentage de valeur caractéristique étant représenté par un rapport entre la valeur caractéristique pour l'une des sources d'éléments nutritifs et une moyenne des valeurs caractéristiques pour toutes les sources d'éléments nutritifs, les pourcentages de valeur caractéristique du sol à évaluer sont comparés aux pourcentages de valeur caractéristique du sol de référence, et ainsi, la labélisation de la région de production pour des produits agricoles est jugée comme fausse dans le cas où une différence entre les deux excède une norme prédéterminée.

Fig. 1

(a)

(b)

(d)

(c)

(e)

Diagnosis results

Soil A:+ + +

Soil B:+

Soil C:+ +

17

EP 2 532 752 B1

Fig. 2

```
                    ( START )
                        |
                        v
   +--------------------------------------+  ~ S0201
   |         Sample creation step         |
   |  +--------------------------------+  |  ~ S0204
   |  |  Soil to be analyzed is obtained| |
   |  +--------------------------------+  |
   |               |                      |
   |               v                      |
   |  +--------------------------------+  |  ~ S0205
   |  |  The aforementioned soil is    |  |
   |  |  suspended in a buffer solution|  |
   |  +--------------------------------+  |
   |               |                      |
   |               v                      |
   |  +--------------------------------+  |  ~ S0206
   |  |  The resultant is diluted to   |  |
   |  |  a predetermined concentration |  |
   |  +--------------------------------+  |
   +--------------------------------------+
                        |
                        v
   +--------------------------------------+  ~ S0202
   |            Dropping step             |
   |  +--------------------------------+  |  ~ S0207
   |  | The created suspension is added|  |
   |  | dropwise to the wells          |  |
   |  +--------------------------------+  |
   +--------------------------------------+
                        |
                        v
   +--------------------------------------+  ~ S0203
   |          Observation step            |
   |  +--------------------------------+  |  ~ S0208
   |  | The rate of consumption for the|  |
   |  | nutrient sources in each well  |  |
   |  | is observed                    |  |
   |  +--------------------------------+  |
   +--------------------------------------+
                        |
                        v
                    (  END  )
```

18

EP 2 532 752 B1

Fig. 3A

```
                    ( START )
                        |
                        v
Regression analysis concerning the plot "Cumulative consumption quantity (yᵢ)      ~S0301a
- Elapsed time (x)" is conducted for the nutrient sources
                        |
                        v
The characteristic values (αᵢ) are acquired based on the                            ~S0302a
regression function for all nutrient sources
                        |
                        v
The characteristic values (αᵢ) are averaged for all nutrient                         ~S0303a
sources, and the average characteristic value (A) is computed
                        |
                        v
The crop cultivation frequency for soil to be analyzed into is                        ~S0304a
measured by using the average characteristic value (A)
                        |
                        v
                    ( END )
```

19

EP 2 532 752 B1

Fig. 3B

```
                    ( START )
                        │
                        ▼
┌─────────────────────────────────────────────────┐
│ Regression analysis concerning the plot "Cumulative consumption │  ── S0301b
│ quantity (y) - Elapsed time (x)"is conducted for the nutrient sources │
└─────────────────────────────────────────────────┘
                        │
                        ▼
┌─────────────────────────────────────────┐
│ The characteristic values (α i) based on  │  ── S0302b
│ the obtained regression function are computed │
└─────────────────────────────────────────┘
                        │
                        ▼
┌─────────────────────────────────────────────────┐
│ The characteristic values (α i) are averaged for all nutrient sources │ ── S0303b
│ and the average characteristic value (A) is computed │
└─────────────────────────────────────────────────┘
                        │
                        ▼
┌─────────────────────────────────────────┐
│ The average information content (E) for the characteristic │ ── S0304b
│ values (α i) is computed │
└─────────────────────────────────────────┘
                        │
                        ▼
┌─────────────────────────────────────────┐
│ The average characteristic value (α i) is │  ── S0305b
│ multiplied by average information content (E) │
│              AE=A×E                          │
└─────────────────────────────────────────┘
                        │
                        ▼
┌─────────────────────────────────────────┐
│ Crop cultivation frequency for the soil to be │ ── S0306b
│ analyzed is measured by using AE │
└─────────────────────────────────────────┘
                        │
                        ▼
                    ( END )
```

20

Fig. 4A

START

The cumulative consumption quantity values (yi, t) of each nutrient source are averaged for all nutrient sources for each predetermined time (Yt)

$$Yt = \sum yi_{,t} / n$$

S0401a

Regression analysis for the plot [Average cumulative consumption quantity (Yt) - Elapsed time (x)] is conducted by using the average cumulative consumption quantity (Yt)

S0402a

The characteristic value (α) is computed based on the regression analysis

S0403a

The crop cultivation frequency of the soil to be analyzed is measured by using characteristic value (α)

S0404a

END

Fig. 4B

START

The cumulative consumption quantity values (yi, t) for each nutrient source is averaged for all nutrient sources (Yt) for each predetermined time and the average value for cumulative consumption quantity (Yt) is computed

$$Y_t = \sum y_{i,t} / n$$

S0401b

The average information content (Et) for the cumulative consumption quantity values (yi, t) is computed for each predetermined time

S0402b

The average value for cumulative consumption quantity (Yt) is multiplied by the average information content (Et)

$$Y_t E_t = Y_t \times E_t$$

S0403b

Using the computed "YtEt", regression analysis of the plot [YtEt – Elapsed time (x)] is conducted

S0404b

The characteristic value ($\alpha$) based on the regression analysis is computed

S0405b

The crop cultivation frequency of the soil to be analyzed is measured by using the characteristic value ($\alpha$)

S0406b

END

Fig. 5A

START

The cumulative consumption quantity values (yi, t) for each nutrient source is averaged for all nutrient sources for each predetermined time $Y_t = \sum y_{i,t} / n$ — S0501a

In regards to the plot [Average cumulative consumption quantity value (Yt) – Elapsed time (x)] from the time "0" until a designated time (T), integral value (S) is computed $S = \int_0^T Y_t \, dx$ — S0502a

The integral value (S) is divided (M) by the elapsed time (T) $M = S / T$ — S0503a

The crop cultivation frequency of the soil to be analyzed is measured by using "M" — S0504a

END

Fig. 5B

$$\text{START}$$

The cumulative consumption quantity values (yi, t) of each nutrient source is averaged for all nutrient sources (Yt) for each predetermined time
$$Y_t = \sum y_{i,t} / n$$

S0501b

The average information content (Et) for the cumulative consumption quantity (yi, t) is computed for each predetermined time

S0502b

The average value (Yt) is multiplied by the average information content (Et)
$$Y_t E_t = Y_t \times E_t$$

S0503b

The integral value (S) for the plot [YtEt – Elapsed time (x)] from the time "0" until a designated time (T) is computed
$$S = \int_0^T Y_{tEt} dx$$

S0504b

The integral value (S) is divided (M) by the elapsed time (T)
$$M = S / T$$

S0505b

The crop cultivation frequency of the soil to be analyzed is measured by using "M"

S0506b

$$\text{END}$$

24

Fig. 6

(a)

| Soil | e | f | k | c |
|------|--------|---------|----------|----------|
| 1071 | 0.9477 | 66.5359 | 221.127  | 122.6364 |
| 1157 | 0.8137 | 33.5773 | 111.6274 | 74.9068  |
| 1433 | 0.7755 | 12.5076 | 26.3581  | 23.3258  |
| 321  | 0.7936 | 12.9401 | 21.8278  | 20.1576  |

(b)

| Soil | e | f | k | c |
|------|--------|---------|----------|----------|
| 1071 | 0.2537 | 9.4489  | 609.8591 | 176.3891 |
| 1157 | 0.6311 | 77.8819 | 225.5781 | 111.4936 |
| 1433 | 0.1254 | 7.5784  | 31.3021  | 23.9626  |
| 321  | 0.1573 | 6.3475  | 25.7585  | 23.5741  |

(c)

| Soil | e | f | k | c |
|------|--------|---------|----------|----------|
| 1071 | 0.8993 | 57.6181 | 187.137  | 112.3473 |
| 1157 | 0.7741 | 31.3442 | 99.301   | 70.8323  |
| 1433 | 0.6962 | 11.1398 | 22.4461  | 20.5952  |
| 321  | 0.7037 | 11.8152 | 19.0915  | 18.146   |

(d)

| Soil | e | f | k | c |
|------|--------|---------|----------|----------|
| 1071 | 1.0593 | 86.5949 | 492.2002 | 167.7242 |
| 1157 | 0.3607 | 19.0541 | 209.943  | 107.6071 |
| 1433 | 0.1168 | 6.2503  | 25.6959  | 20.8781  |
| 321  | 0.1485 | 4.7951  | 20.2018  | 19.1977  |

Fig. 7

(a)

| Soil | a | c |
|---|---|---|
| 1071 | 1.0228 | 167.5455 |
| 1157 | 0.7219 | 123.4361 |
| 1433 | 0.1651 | 30.1626 |
| 321 | 0.2411 | 40.5047 |

(b)

| Soil | a | c |
|---|---|---|
| 1071 | 1.0228 | 167.5455 |
| 1157 | 0.7219 | 123.4361 |
| 1433 | 0.1651 | 30.1626 |
| 321 | 0.2411 | 40.5047 |

(c)

| Soil | a | c |
|---|---|---|
| 1071 | 0.9778 | 161.259 |
| 1157 | 0.6922 | 119.7267 |
| 1433 | 0.1483 | 27.761 |
| 321 | 0.2079 | 36.0985 |

(d)

| Soil | a | c |
|---|---|---|
| 1071 | 0.9868 | 161.1658 |
| 1157 | 0.701 | 119.7038 |
| 1433 | 0.1538 | 27.555 |
| 321 | 0.2156 | 36.0497 |

Fig. 8

(a)

| Soil | a | c |
|------|--------|----------|
| 1071 | 0.8199 | 154.9654 |
| 1157 | 0.6194 | 117.08 |
| 1433 | 0.1568 | 29.6529 |
| 321 | 0.2012 | 38.0273 |

(b)

| Soil | a | c |
|------|--------|----------|
| 1071 | 0.8199 | 154.9654 |
| 1157 | 0.6194 | 117.08 |
| 1433 | 0.1568 | 29.6529 |
| 321 | 0.2012 | 38.0273 |

(c)

| Soil | a | c |
|------|--------|----------|
| 1071 | 0.7914 | 149.5746 |
| 1157 | 0.6035 | 114.0704 |
| 1433 | 0.1448 | 27.3821 |
| 321 | 0.1817 | 34.3503 |

(d)

| Soil | a | c |
|------|--------|----------|
| 1071 | 0.7872 | 148.7924 |
| 1157 | 0.6003 | 113.4574 |
| 1433 | 0.1418 | 26.8119 |
| 321 | 0.1786 | 33.7557 |

Fig. 9

(a)

| Soil | b |
|------|---------|
| 1071 | 71.3997 |
| 1157 | 55.5691 |
| 1433 | 14.6418 |
| 321  | 17.8356 |

(b)

| Soil | b |
|------|---------|
| 1071 | 71.3997 |
| 1157 | 55.5691 |
| 1433 | 14.6418 |
| 321  | 17.8356 |

(c)

| Soil | b |
|------|---------|
| 1071 | 69.0799 |
| 1157 | 54.3363 |
| 1433 | 13.4915 |
| 321  | 16.2795 |

(d)

| Soil | b |
|------|---------|
| 1071 | 68.4036 |
| 1157 | 53.8049 |
| 1433 | 13.0933 |
| 321  | 15.7831 |

EP 2 532 752 B1

Fig. 10

29

Fig. 11

```
                         ┌──────────────────────────┐
                         │           START          │
                         └──────────────────────────┘
                                       ↓
   ┌──────────────────────────────────────────────────────────────┐   S1101
   │      Benchmark soil is collected from specific agricultural land │
   └──────────────────────────────────────────────────────────────┘   S1102
                                       ↓
   ┌──────────────────────────────────────────────────────────────┐   S1106
   │    ┌────────────────────────────────────────────────────┐    │
   │    │      Benchmark soil is suspended in the buffer solution │    │
   │    └────────────────────────────────────────────────────┘    │
   │                             ↓                                  │   S1107
   │    ┌────────────────────────────────────────────────────┐    │
   │    │  The suspended solution is diluted up to a predetermined concentration │
   │    └────────────────────────────────────────────────────┘    │
   │                             ↓                                  │   S1108
   │    ┌────────────────────────────────────────────────────┐    │
   │    │        The diluted solution is added dropwise to        │
   │    │         the wells containing nutrient sources           │
   │    └────────────────────────────────────────────────────┘    │
   │                             ↓                                  │   S1109
   │    ┌────────────────────────────────────────────────────┐    │
   │    │  The rate of consumption of the nutrient sources is observed │
   │    └────────────────────────────────────────────────────┘    │
   │                             ↓                                  │   S1110
   │    ┌────────────────────────────────────────────────────┐    │
   │    │  The crop cultivation frequency for the benchmark soil is measured │
   │    └────────────────────────────────────────────────────┘    │
   └──────────────────────────────────────────────────────────────┘
                                       ↓
   ┌──────────────────────────────────────────────────────────────┐   S1103
   │    Soil attached to the agricultural products for which tests are │
   │    conducted is collected                                       │
   └──────────────────────────────────────────────────────────────┘
                                       ↓                                S1104
   ┌──────────────────────────────────────────────────────────────┐
   │    ┌────────────────────────────────────────────────────┐    │
   │    │      Soil to be tested is suspended in the buffer solution │    │
   │    └────────────────────────────────────────────────────┘    │
   │                             ↓                                  │
   │    ┌────────────────────────────────────────────────────┐    │
   │    │  The suspended solution is diluted up to a predetermined concentration │
   │    └────────────────────────────────────────────────────┘    │
   │                             ↓                                  │
   │    ┌────────────────────────────────────────────────────┐    │
   │    │        The diluted solution is added dropwise to        │
   │    │         the wells containing nutrient sources           │
   │    └────────────────────────────────────────────────────┘    │
   │                             ↓                                  │
   │    ┌────────────────────────────────────────────────────┐    │
   │    │  The rate of consumption of the nutrient sources is observed │
   │    └────────────────────────────────────────────────────┘    │
   │                             ↓                                  │
   │    ┌────────────────────────────────────────────────────┐    │
   │    │  The crop cultivation frequency for the soil to analyzed is measured │
   │    └────────────────────────────────────────────────────┘    │
   └──────────────────────────────────────────────────────────────┘
                                       ↓                                S1105
   ┌──────────────────────────────────────────────────────────────┐
   │  Based on comparison of the crop cultivation frequency of the soil to be │
   │  tested and that of the benchmark soil, and in case that the difference │
   │  between the two exceeds a predetermined standard, the labeling of the │
   │  production area of the agricultural products is judged as being deceptive │
   └──────────────────────────────────────────────────────────────┘
                                       ↓
                         ┌──────────────────────────┐
                         │            END           │
                         └──────────────────────────┘
```

Fig. 12

```
                              ┌─────────────────┐
                              │      START      │
                              └─────────────────┘
                                       ↓
   ┌───────────────────────────────────────────────────────────────┐          S1201
   │   Benchmark soil is collected from specific agricultural land   │
   └───────────────────────────────────────────────────────────────┘
                                       ↓                                        S1202
   ┌───────────────────────────────────────────────────────────────┐          S1206
   │  ┌─────────────────────────────────────────────────────────┐  │
   │  │      Benchmark soil is suspended in the buffer solution   │  │
   │  └─────────────────────────────────────────────────────────┘  │
   │                            ↓                                    │          S1207
   │  ┌─────────────────────────────────────────────────────────┐  │
   │  │ The suspended solution is diluted up to a predetermined concentration │
   │  └─────────────────────────────────────────────────────────┘  │
   │                            ↓                                    │          S1208
   │  ┌─────────────────────────────────────────────────────────┐  │
   │  │           The diluted solution is added dropwise to        │  │
   │  │           the wells containing nutrient sources            │  │
   │  └─────────────────────────────────────────────────────────┘  │
   │                            ↓                                    │          S1209
   │  ┌─────────────────────────────────────────────────────────┐  │
   │  │  The rate of consumption of the nutrient sources is observed │
   │  └─────────────────────────────────────────────────────────┘  │
   │                            ↓                                    │          S1210
   │  ┌─────────────────────────────────────────────────────────┐  │
   │  │  Characteristics values for each nutrient source are computed │
   │  └─────────────────────────────────────────────────────────┘  │
   └───────────────────────────────────────────────────────────────┘
                                       ↓
   ┌───────────────────────────────────────────────────────────────┐          S1203
   │   Soil attached to the agricultural products for which tests are │
   │   conducted is collected                                        │
   └───────────────────────────────────────────────────────────────┘
                                       ↓                                        S1204
   ┌───────────────────────────────────────────────────────────────┐
   │  ┌─────────────────────────────────────────────────────────┐  │
   │  │      Soil to be tested is suspended in the buffer solution  │  │
   │  └─────────────────────────────────────────────────────────┘  │
   │                            ↓                                    │
   │  ┌─────────────────────────────────────────────────────────┐  │
   │  │ The suspended solution is diluted up to a predetermined concentration │
   │  └─────────────────────────────────────────────────────────┘  │
   │                            ↓                                    │
   │  ┌─────────────────────────────────────────────────────────┐  │
   │  │           The diluted solution is added dropwise to        │  │
   │  │           the wells containing nutrient sources            │  │
   │  └─────────────────────────────────────────────────────────┘  │
   │                            ↓                                    │
   │  ┌─────────────────────────────────────────────────────────┐  │
   │  │  The rate of consumption of the nutrient sources is observed │
   │  └─────────────────────────────────────────────────────────┘  │
   │                            ↓                                    │
   │  ┌─────────────────────────────────────────────────────────┐  │
   │  │  Characteristics values for all nutrient source are computed │
   │  └─────────────────────────────────────────────────────────┘  │
   └───────────────────────────────────────────────────────────────┘
                                       ↓                                        S1205
   ┌───────────────────────────────────────────────────────────────┐
   │ Characteristic value percentages are computed for the nutrient sources in the │
   │ soil to be tested and the benchmark soil, the characteristic value percentages │
   │ for the soil to be tested are compared with the characteristic value for the   │
   │ benchmark soil, and thereby, the labeling of production region for the         │
   │ agricultural products is judged to be false in case that a difference between  │
   │ the two exceeds a predetermined standard                                       │
   └───────────────────────────────────────────────────────────────┘
                                       ↓
                              ┌─────────────────┐
                              │       END       │
                              └─────────────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2007000138 A

• JP 2006329639 A

**Non-patent literature cited in the description**

• **YAO, H. et al.** Microbial Biomass and Community Structure in a Sequence of Soils with Increasing Fertility and Changing Land Use. *Microbial Ecology,* 01 October 2000, vol. 40, 223-237

• **SATO, NOBUYOSHI et al.** Distributed Geographical Origin Identification System for Green-groceries by Trace Element Compositions. *TRANSACTIONS OF INFORMATION PROCESSING SOCIETY OF JAPAN,* 15 July 2006, vol. 47 (7), 2151-2159